# EUROPEAN PATENT SPECIFICATION

(11) **EP 3 932 971 B1**
(45) Date of publication and mention of the grant of the patent: **16.04.2025**
(21) Application number: 20763281.1
(22) Date of filing: 19.02.2020
(51) Int. Cl.: C08G 59/50, C08G 59/68, C09J 11/06, C09J 163/02, C09J 163/04, A61B 1/00, C08K 5/057

(54) **ADHESIVE FOR ENDOSCOPE, CURED PRODUCT THEREOF, AND ENDOSCOPE AND MANUFACTURING METHOD THEREOF**
KLEBSTOFF FÜR ENDOSKOP, GEHÄRTETES PRODUKT DAVON SOWIE ENDOSKOP UND VERFAHREN ZU SEINER HERSTELLUNG
ADHÉSIF POUR ENDOSCOPE, PRODUIT DURCI DE CELUI-CI, ET ENDOSCOPE ET SON PROCÉDÉ DE FABRICATION

(30) Priority: 26.02.2019 JP 2019032974
(43) Date of publication of application: 05.01.2022
(73) Proprietor: FUJIFILM Corporation, Tokyo 106-8620 (JP)
(72) Inventor: NAKAI, Yoshihiro, Ashigarakami-gun, Kanagawa 258-8577 (JP); FURUKAWA, Kazushi, Ashigarakami-gun, Kanagawa 258-8577 (JP)
(74) Representative: HGF
(86) International application number: PCT/JP2020/006543
(87) International publication number: WO 2020/175278

(56) References cited:
- WO-A1-2017/204012
- WO-A1-2017/204012
- WO-A1-2018/105056
- WO-A1-2020/013003
- JP-A- 2001 059 013
- JP-A- 2001 059 013
- JP-A- 2001 288 244
- JP-A- 2001 288 244
- JP-A- 2004 231 869
- JP-A- 2004 231 869
- JP-A- 2005 036 080
- JP-A- 2005 036 080
- JP-A- 2015 117 283
- JP-A- 2016 130 307
- JP-A- 2016 130 307
- JP-A- H08 100 107

## Description

### BACKGROUND OF THE INVENTION

### 1. Field of the Invention

The present invention relates to adhesives for endoscopes, cured products thereof, endoscopes, and methods for manufacturing endoscopes.

### 2. Description of the Related Art

Endoscopes for observation of body cavities, the gastrointestinal tract, the esophagus, and other parts of a human body are repeatedly used. Therefore, the flexible tube that forms the insertion section of an endoscope is cleaned and disinfected with a chemical after each use. In particular, a cleanliness level that ensures sterilization beyond disinfection is required for insertion into a site where there is a high risk of infection, such as a bronchus. Accordingly, there is a need for an endoscope having high durability sufficient to withstand repeated disinfection or sterilization treatment.

The insertion section of an endoscope is inserted through the mouth or the nose into the body. It is desirable to reduce the diameter of the insertion section of an endoscope to alleviate discomfort and pain experienced by patients during insertion. Accordingly, adhesives are mainly used instead of bulky members such as screws to join members that form the insertion section.

Among adhesives, epoxy-based adhesives are used to bond endoscope constituent members because these adhesives have high workability and cured products thereof have superior properties such as high adhesiveness, heat resistance, and moisture resistance.

For example, JP2002-238834A discloses an endoscope device in which parts that form the endoscope are joined together with a two-component reactive adhesive obtained by mixing together a base material composed of an epoxy resin having 1% to 50% by weight of rubber and/or plastic added thereto and a curing agent composed of at least one amine selected from the group consisting of aliphatic amines, polyamide-amines, aromatic amines, cyclic amines, and aliphatic-aromatic amines in a ratio of 10:1 to 10:7. According to JP2002-238834A, this endoscope does not exhibit decreased adhesion strength when subjected to various disinfection processes and can maintain its performance over a long period of time. WO 2017/204012 A1 relates to an adhesive composition, an ultrasonic transducer, an endoscope apparatus, and an ultrasound endoscopy device comprising epoxy resin as main component and inorganic zwitterion exchanger.

### SUMMARY OF THE INVENTION

Because endoscopes are repeatedly used over a long period of time, it is required that a state in which an endoscope member is secured with an adhesive can be sufficiently maintained after repeated use over a long period of time. That is, it is required that the secured state can be sufficiently maintained after repeated exposure to wet conditions during insertion into the body or immersion in disinfectant solution. It is also important to have durability so that the secured state can be sufficiently maintained after repeated exposure to sterilization treatment. Unfortunately, after conducting research on epoxy-based adhesives in the related art, including that disclosed in the above patent document, the inventors have found that these adhesives fail to simultaneously achieve wet durability and sterilization durability required for endoscope applications at a sufficiently high level.

An object of the present invention is to provide an adhesive for endoscopes that is suitable for securing an endoscope constituent member and that can maintain sufficient adhesiveness after extended exposure to wet conditions or after repeated exposure to sterilization treatment in a state in which the member is secured with the adhesive (in the form of a cured product), and also to provide a cured product of such an adhesive. Another object of the present invention is to provide an endoscope that exhibits less decrease in performance after extended exposure to wet conditions or after repeated exposure to sterilization treatment, and also to provide a method for manufacturing such an endoscope.

After conducting intensive research in view of the foregoing problem, the inventors have found that, if an epoxy-based adhesive contains an epoxy resin as a base material in combination with a particular polyamine compound as a curing component and further contains a metal alkoxide compound, a secured state in which a member is joined with the adhesive can be sufficiently maintained after extended exposure to wet conditions or after repeated exposure to sterilization treatment. The present invention has been made based on these findings and further research.

The foregoing objects of the present invention have been achieved by the following solutions.
[1] An adhesive for an endoscope, including the following (a) to (c):
   (a) an epoxy resin including at least one of a bisphenol A epoxy resin, a bisphenol F epoxy resin, or a phenol novolac epoxy resin;
   (b) a polyamine compound having an oxygen atom but no amide bond in a molecule thereof; and
   (c) a metal alkoxide compound;
   wherein the polyamine compound has an oxyalkylene structure.
[2] The adhesive for an endoscope according to [1], wherein the adhesive for an endoscope includes a silicon alkoxide compound as the metal alkoxide compound.
[3] The adhesive for an endoscope according to any one of [1] to [2], wherein the adhesive for an endoscope includes a silicon alkoxide compound having an oxirane ring as the metal alkoxide compound.
[4] The adhesive for an endoscope according to any one of [1] to [3], wherein the adhesive for an endoscope includes, as the metal alkoxide compound, a silicon alkoxide compound having an aliphatic ring having an oxirane ring fused thereto.
[5] The adhesive for an endoscope according to any one of [1] to [4], wherein the adhesive for an endoscope includes, as the metal alkoxide compound, at least one of a titanium alkoxide compound, an aluminum alkoxide compound, or a zirconium alkoxide compound.
[6] The adhesive for an endoscope according to [5], wherein the titanium alkoxide compound includes an atom of at least one of N, P, or S or has an acetato structure.
[7] The adhesive for an endoscope according to [5], wherein the aluminum alkoxide compound includes at least one of an acetonato structure or an acetato structure.
[8] The adhesive for an endoscope according to [5], wherein the zirconium alkoxide compound includes at least one of an acetonato structure, an acetato structure, or a lactato structure.
[9] A cured product obtained by curing the adhesive for an endoscope according to any one of [1] to [8].
[10] An endoscope including a constituent member secured with the cured product according to [9].
[11] A method for manufacturing an endoscope, including securing a constituent member with the adhesive for an endoscope according to any one of [1] to [8].

In the description of the present invention, a numerical range represented by "to" is meant to include values recited before and after "to" as lower and upper limits.

In the present specification, when a substituent is not explicitly specified as substituted or unsubstituted (the same is true for linking groups), it is meant that the group may have any substituent as long as the desired effect is achieved. The same is true for compounds that are not explicitly specified as substituted or unsubstituted.

In the present specification, when the number of carbon atoms in a certain group is specified, the number of carbon atoms refers to the number of carbon atoms in the entire group. That is, when the group further has a substituent, the number of carbon atoms refers to the number of carbon atoms in the entire group including the substituent.

The adhesive for an endoscope according to the present invention can maintain sufficient adhesiveness after extended exposure to wet conditions or after repeated exposure to sterilization treatment in a state in which an endoscope member is secured with the adhesive (in the form of a cured product). In addition, the cured product according to the present invention has high long-term wet durability and also has high durability against repeated sterilization treatment. In addition, the endoscope according to the present invention exhibits less decrease in performance after extended exposure to wet conditions or after repeated exposure to sterilization treatment. Furthermore, the method for manufacturing an endoscope according to the present invention can provide an endoscope that exhibits less decrease in performance after extended exposure to wet conditions or after repeated exposure to sterilization treatment.

### BRIEF DESCRIPTION OF THE DRAWINGS

FIG. 1 is an external view illustrating the configuration of an embodiment of an endoscope according to the present invention;
FIG. 2 is a partial sectional view illustrating the configuration of an insertion section of the endoscope illustrated in FIG. 1;
FIG. 3 is an external perspective view of a tip portion of the insertion section; and
FIG. 4 is a partially cutaway partial sectional view of the tip portion, in which hatching indicating cross-sections of lenses and a prism is omitted.

### DESCRIPTION OF THE PREFERRED EMBODIMENTS

### Adhesive for Endoscope

A preferred embodiment of an adhesive for an endoscope according to the present invention will now be described.

The adhesive for an endoscope according to the present invention (hereinafter also referred to as "adhesive according to the present invention") includes the following components (a) to (c):
(a) an epoxy resin including at least one of a bisphenol A epoxy resin, a bisphenol F epoxy resin, or a phenol novolac epoxy resin;
(b) a polyamine compound having an oxygen atom but no amide bond in the molecule thereof; and
(c) a metal alkoxide compound.

The epoxy resin (a) (hereinafter also simply referred to as "component (a)) is a base material for the adhesive. The polyamine compound (b) (hereinafter also simply referred to as "component (b)) is a curing component that reacts with the epoxy resin to cure the adhesive. In addition to the base material and the curing component, the adhesive according to the present invention contains the metal alkoxide compound (c) (hereinafter also simply referred to as "component (c)).

The form of the adhesive according to the present invention is not limited as long as it includes the above components. For example, the adhesive for an endoscope according to the present invention may contain a mixture of the components (a) to (c) (one-component type) or may include the components (a) to (c) in a state in which one of the components (a) to (c) is separated from the other components (two-component type). Alternatively, the adhesive for an endoscope according to the present invention may include the components (a) to (c) in a state in which the components (a) to (c) are separated from each other (three-component type). The adhesive according to the present invention encompasses all of these forms.

When the amounts of the components present in the adhesive are described in the present specification, or when the amounts of the components present in the adhesive are specified in the present invention, it is meant, in the case of a form such as a two-component type or a three-component type, that the components (a) to (c) are mixed together before use such that the individual components are present in the mixture in the desired amounts described or specified as above. That is, the individual components (a) to (c) do not have to be present in the amounts described in the present specification or specified in the present invention in a state in which the components are separated. In other words, in the case of a form such as a two-component type or a three-component type, it is meant that the components (a) to (c) are present in the amounts described in the present specification or specified in the present invention after the components (a) to (c) are mixed together before use.

If the adhesive for an endoscope according to the present invention is of a one-component type or is of a two-component or other type in which components that can react with each other have been mixed together (e.g., if the epoxy resin and the polyamine compound have been mixed together), the adhesive is preferably stored at a low temperature at which practically no reaction occurs in order to ensure that the components are stably maintained with no or sufficiently inhibited reaction with each other. For example, the adhesive can be stored at -20°C or lower, preferably -30°C or lower, more preferably -40°C or lower, even more preferably -50°C or lower. If necessary, light can be blocked during storage.

The adhesive according to the present invention may include, for example, solvents, plasticizers, adhesiveness enhancers (e.g., silane coupling agents), surfactants, colorants (e.g., pigments and dyes), weathering agents, antioxidants, heat stabilizers, lubricants, antistatic agents, whiteners, release agents, conductors, viscosity modifiers, fillers (e.g., silica and calcium carbonate), thixotropic agents, diluents, and flame retardants as long as they do not interfere with the advantages of the present invention.

A cured product obtained by curing the adhesive according to the present invention can maintain sufficient adhesiveness after extended exposure to wet conditions or after repeated exposure to sterilization treatment. Although the mechanism is not fully understood, it can be attributed to, for example, the combined effect of the following factors: the component (b) has an oxygen atom but no amide bond in the molecule thereof and thus imparts moderate flexibility to the cured product so that it becomes tougher, and the component (c) forms chemical bonding at the bonded interface or reacts or interacts with other components.

The adhesive according to the present invention is suitable for securing various members that form endoscopes (endoscope constituent members). That is, the adhesive according to the present invention is suitable for use in bonding (joining) and securing an endoscope constituent member to another endoscope constituent member. The adhesive used for securing the endoscope constituent member becomes a cured product that forms a bonded region of the endoscope.

The member secured with the adhesive according to the present invention is not particularly limited. Examples of preferred members include metal members, glass members, and resin members. The endoscope constituent member is "secured" by bonding the endoscope constituent member to another member that forms the endoscope (support member). The support member may be a tube wall or other portion of the endoscope or an immovable member secured thereto, or may be a member, such as a tube, whose relative position can be changed within the endoscope. In the present invention, the term "secure" is meant to include filling, i.e., sealing, the space between the endoscope constituent member and the support member to which the member is to be joined with a cured product of the adhesive.

The individual components that form the adhesive according to the present invention will hereinafter be described.

### (a) Epoxy Resin

The adhesive according to the present invention includes an epoxy resin as the component (a). The epoxy resin includes at least one of a bisphenol A epoxy resin, a bisphenol F epoxy resin, or a phenol novolac epoxy resin. The adhesive according to the present invention may include one or more epoxy resins selected from the group consisting of bisphenol A epoxy resins, bisphenol F epoxy resins, and phenol novolac epoxy resins.

The proportion of the total amount of the bisphenol A epoxy resin, the bisphenol F epoxy resin, and the phenol novolac epoxy resin to the total amount of the epoxy resin present in the adhesive according to the present invention is preferably 70% by mass or more, more preferably 80% by mass or more, even more preferably 90% by mass or more. More preferably, the epoxy resin present in the adhesive according to the present invention is at least one of a bisphenol A epoxy resin, a bisphenol F epoxy resin, or a phenol novolac epoxy resin.

The epoxy equivalent weight of the epoxy resin present in the adhesive according to the present invention is preferably 10 to 1,000, more preferably 50 to 500, even more preferably 80 to 400, particularly preferably 100 to 300. The epoxy resin present in the adhesive according to the present invention typically has two or more epoxy groups per molecule.

The epoxy equivalent weight is determined by dividing the molecular weight of the epoxy compound by the number of moles of epoxy groups in the epoxy compound.

The bisphenol A epoxy resin that can be used in the adhesive according to the present invention is not particularly limited, and a wide range of bisphenol A epoxy resins commonly used as base materials for epoxy-based adhesives can be used. Specific examples of preferred bisphenol A epoxy resins include bisphenol A diglycidyl ethers (jER 825, jER 828, and jER 834 (all of which are trade names), manufactured by Mitsubishi Chemical Corporation) and bisphenol A propoxylate diglycidyl ethers (manufactured by Sigma-Aldrich Co.).

The bisphenol F epoxy resin that can be used in the adhesive according to the present invention is not particularly limited, and a wide range of bisphenol F epoxy resins commonly used as base materials for epoxy-based adhesives can be used. Specific examples of preferred bisphenol F epoxy resins include bisphenol F diglycidyl ethers (trade name: EPICLON 830, manufactured by DIC Corporation) and 4,4'-methylenebis(N,N-diglycidylaniline).

The phenol novolac epoxy resin that can be used in the adhesive according to the present invention is not particularly limited, and a wide range of phenol novolac epoxy resins commonly used as base materials for epoxy-based adhesives can be used. An example of such a phenol novolac epoxy resin is sold as Product No. 406775 from Sigma-Aldrich Co.

The amount of the epoxy resin present in the adhesive according to the present invention may be, for example, 5% to 90% by mass, more preferably 10% to 90% by mass. The amount of the epoxy resin present in the adhesive is also preferably 20% to 90% by mass, or preferably 30% to 90% by mass, or preferably 40% to 90% by mass, or preferably 50% to 85% by mass, or preferably 60% to 85% by mass.

### (b) Polyamine Compound

The adhesive according to the present invention contains one or more polyamine compounds as the component (b). The polyamine compound serving as the component (b) has an oxygen atom in the molecule thereof. In addition, the polyamine compound serving as the component (b) has no amide bond (-NH-CO-) in the molecule thereof, which distinguishes the polyamine compound from polyamide-amines. The polyamine compound serving as the component (b) is a compound having two or more amino groups having an active hydrogen per molecule. The polyamine compound preferably has an unsubstituted amino group (-NH₂), more preferably two or more unsubstituted amino groups. Even more preferably, the polyamine compound is a primary polyamine compound (i.e., a polyamine compound in which all amino groups are unsubstituted amino groups).

The polyamine compound serving as the component (b) preferably has 2 to 10, more preferably 2 to 8, even more preferably 2 to 6, still more preferably 2 to 4, particularly preferably 2 or 3, amino groups having an active hydrogen per molecule. In particular, at least one selected from the group consisting of diamine compounds and triamine compounds is suitable for use as the polyamine compound.

The active hydrogen equivalent weight (equivalent weight per active hydrogen in amino groups) of the polyamine compound serving as the component (b) is preferably 10 to 2,000, more preferably 20 to 1,000, even more preferably 30 to 900, still more preferably 40 to 800, still even more preferably 60 to 700, particularly preferably 65 to 600.

The active hydrogen equivalent weight is determined by dividing the molecular weight of the polyamine compound by the number of moles of active hydrogens in the amino groups of the polyamine compound (which means the molecular weight of the polyamine compound per active hydrogen in the amino groups).

The molecular weight of the polyamine compound serving as the component (b) is preferably 100 to 6,000, more preferably 100 to 3,000. If the polyamine compound is a polymer (e.g., if the polyamine compound has a polyoxyalkylene group, as described later), the molecular weight refers to number average molecular weight.

In particular, the polyamine compound serving as the component (b) has an oxyalkylene structure, more preferably a polyoxyalkylene structure, in the molecule thereof to impart higher flexibility to the cured product so that it becomes tougher.

More preferably, the polyamine compound having an oxyalkylene structure is a polyoxyalkylenediamine compound or a polyoxyalkylenetriamine compound.

The alkylene group of the oxyalkylene structure may be a linear alkylene group or a branched alkylene group. The alkylene group of the oxyalkylene structure preferably has 1 to 10 carbon atoms, more preferably 2 to 6 carbon atoms, even more preferably 2 to 4 carbon atoms.

More preferably, the oxyalkylene structure is an oxyethylene structure or an oxypropylene structure.

If the polyamine compound serving as the component (b) has a polyoxyalkylene structure, the plurality of oxyalkylene groups that form the polyoxyalkylene structure may be the same as or different from each other. The average number of repeating units of oxyalkylene groups in the polyoxyalkylene structure is preferably 2 to 1,000, more preferably 3 to 500. The average number of repeating units is also preferably 2 to 100, or preferably 2 to 50, or preferably 2 to 35, or preferably 2 to 25. The polyamine compound serving as the component (b) may have a plurality of polyoxyalkylene structures.

Specific examples of preferred polyamine compounds that can be used in the present invention are given below. Numbers after parentheses are the average numbers of repeating units in the parentheses.

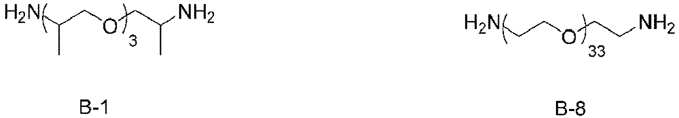

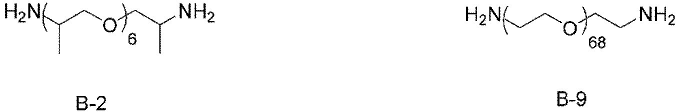

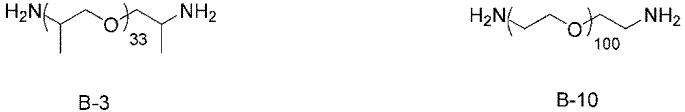

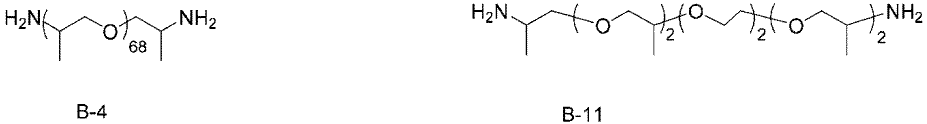

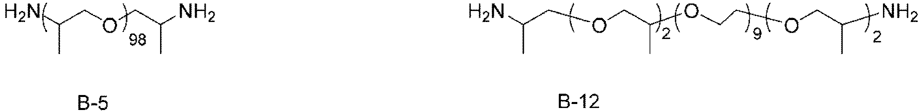

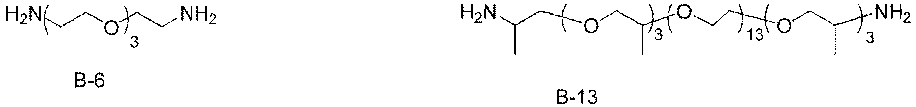

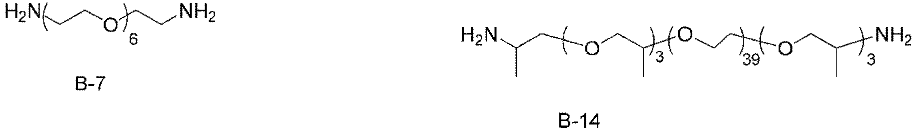

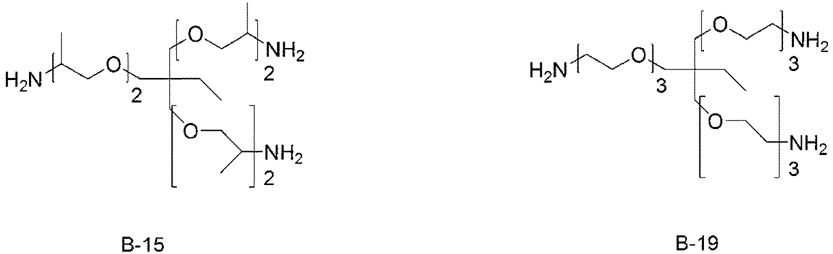

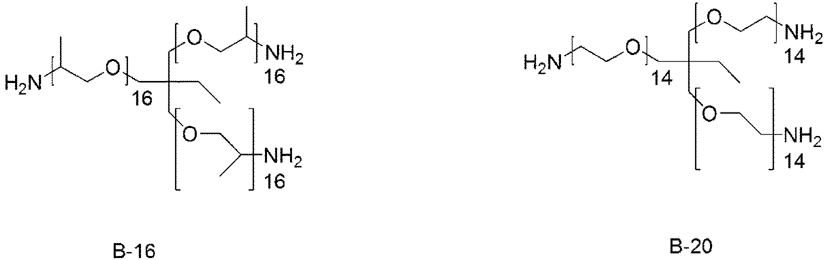

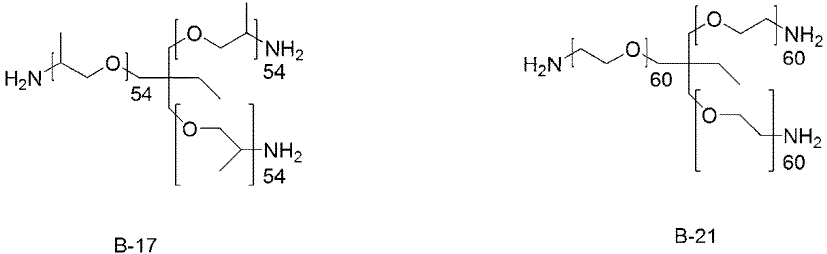

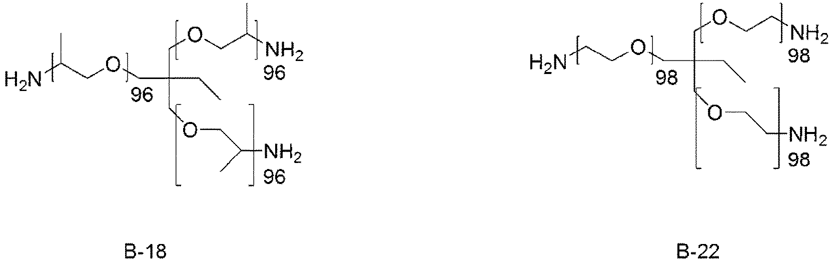

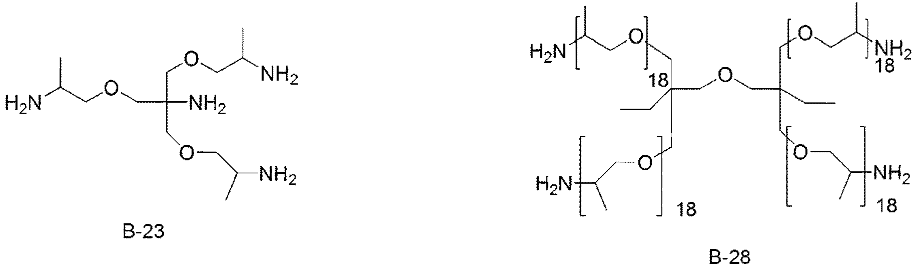

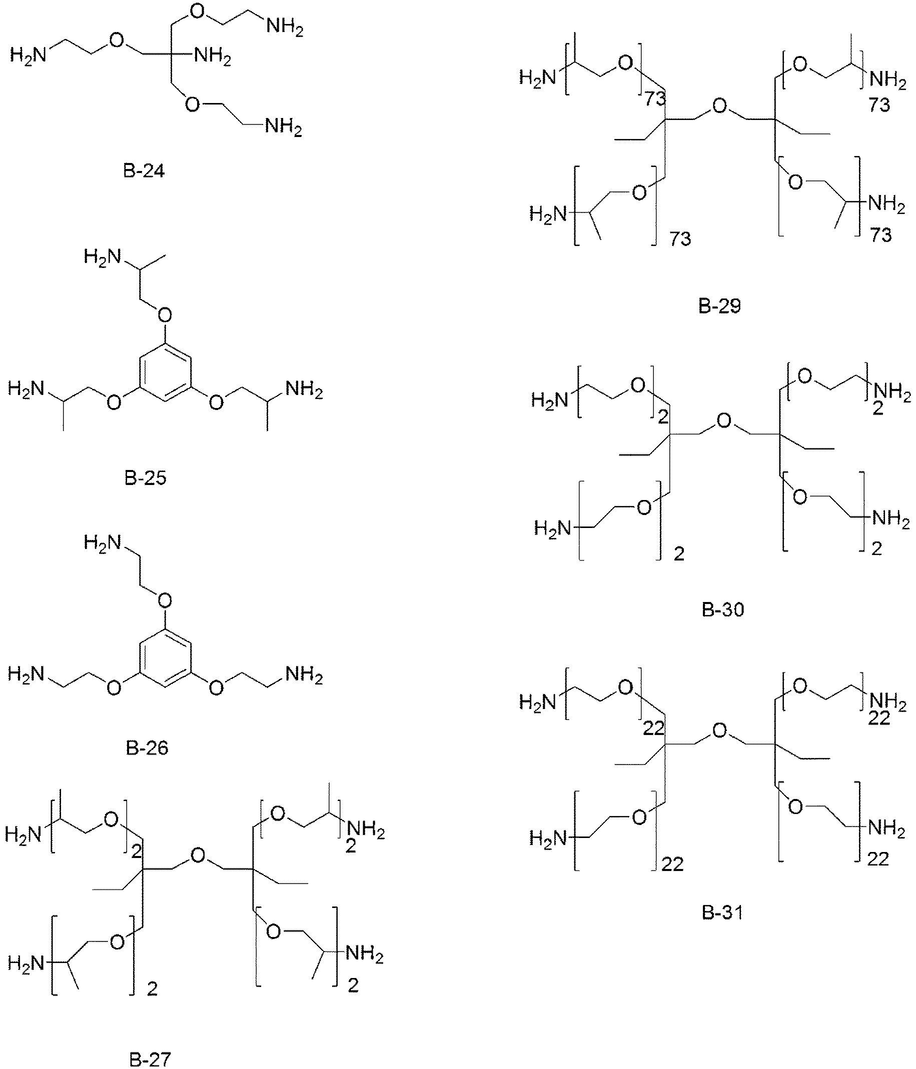

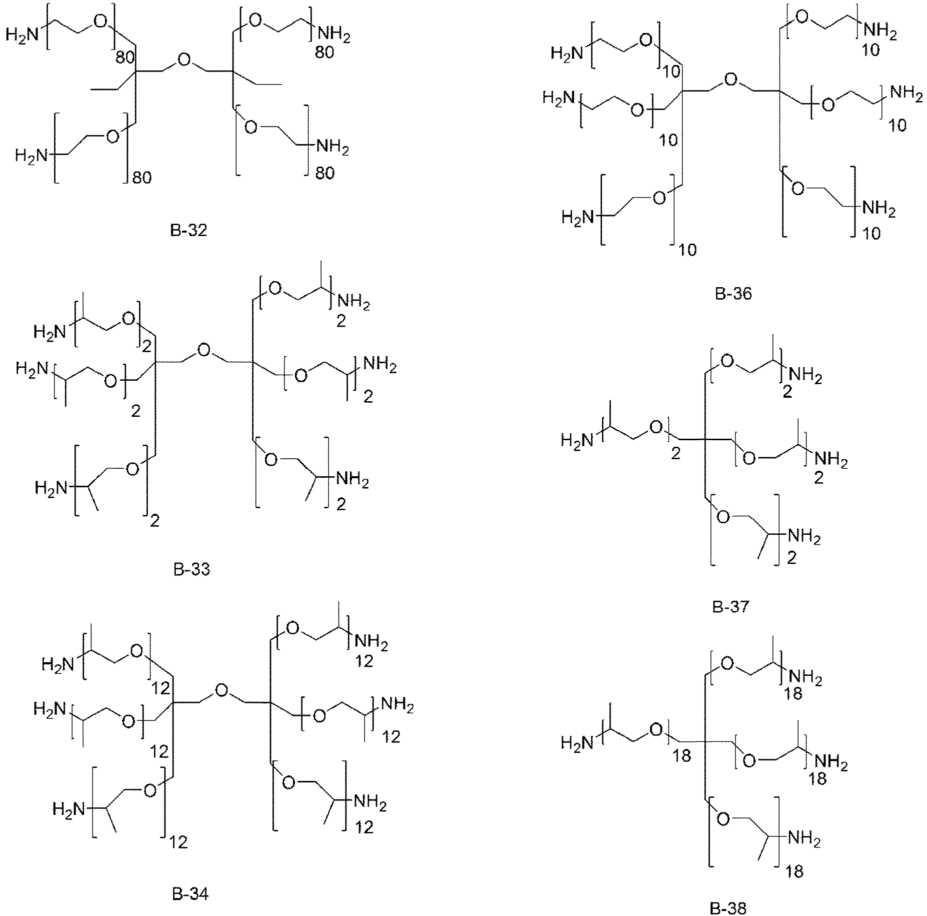

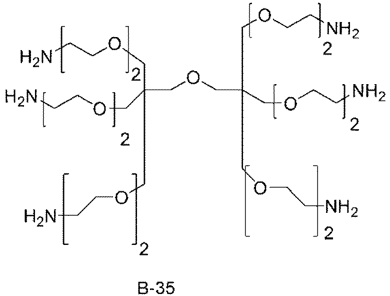

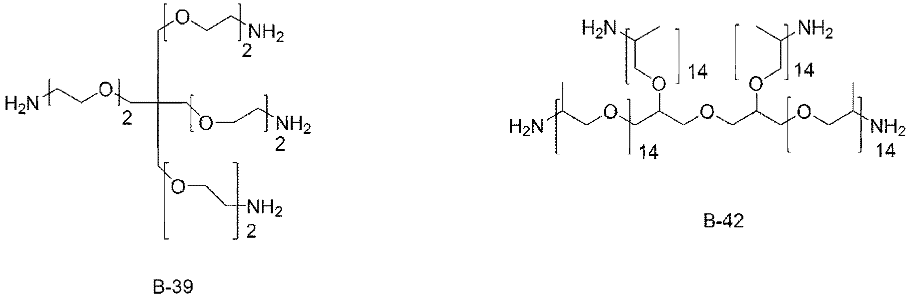

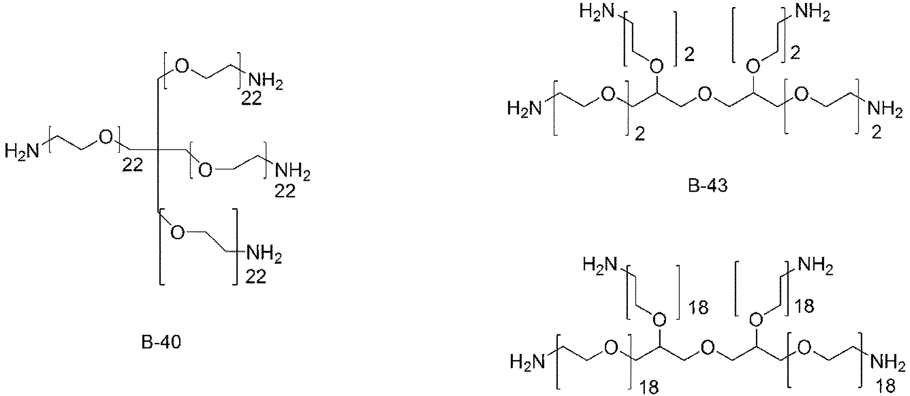

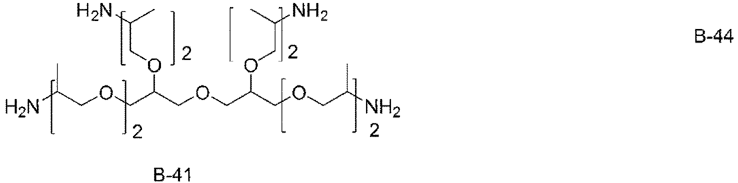

The polyamine compound serving as the component (b) can be synthesized as usual. Commercial products may also be used.

The amount of the polyamine compound serving as the component (b) present in the adhesive according to the present invention may be appropriately set by taking into account, for example, the active hydrogen equivalent weight and the molecular weight. For example, the amount of the polyamine compound serving as the component (b) may be 5 to 300 parts by mass, more preferably 10 to 250 parts by mass, even more preferably 15 to 220 parts by mass, based on 100 parts by mass of the epoxy resin. The amount of the polyamine compound serving as the component (b) is also preferably 5 to 200 parts by mass, or preferably 10 to 150 parts by mass, or preferably 10 to 100 parts by mass, or preferably 15 to 70 parts by mass, or preferably 15 to 50 parts by mass, based on 100 parts by mass of the epoxy resin. The ratio of the active hydrogen equivalent weight of the polyamine compound to the epoxy equivalent weight of the epoxy resin (active hydrogen equivalent weight/epoxy equivalent weight) is preferably 0.05 to 1.5, more preferably 0.05 to 1.2, even more preferably 0.06 to 1.0.

The adhesive according to the present invention may contain a curing component other than the polyamine compound serving as the component (b). The proportion of the polyamine compound serving as the component (b) to all curing component is preferably 80% by mass or more, more preferably 90% by mass or more. It is also preferred that all curing component be the polyamine compound serving as the component (b). If the adhesive according to the present invention contains a curing component other than the polyamine compound, various curing agents and curing aids known as curing components for epoxy-based adhesives can be used as the curing component. For example, the polyamine compound can be used in combination with at least one of an acid anhydride-based compound, an imidazole-based compound, a phosphorus-based compound, a thiol-based compound, a dicyandiamide-based compound, or a phenol-based compound.

### (c) Metal Alkoxide Compound

The adhesive according to the present invention contains one or more metal alkoxide compounds as the component (c). In the present invention, "metal alkoxide compound" refers to a compound having a structure in which at least one alkoxy group is attached to a metal atom. The alkoxy group may have a substituent. The substituent may be monovalent or divalent (e.g., an alkylidene group). In addition, two alkoxy groups attached to one metal atom may be attached to each other to form a ring.

Examples of metals that form the metal alkoxide compound serving as the component (c) include Si, Al, B, Ba, Bi, Ca, Ga, Ge, Hf, In, La, Mg, Nb, P, Sr, Sn, Ta, Ti, V, Y, and Zr. Particularly preferred are Si, Ti, Al, and Zr.

The adhesive according to the present invention preferably includes a silicon alkoxide compound as the metal alkoxide compound. It is also preferred that the adhesive according to the present invention include, instead of or in addition to the silicon alkoxide compound, at least one of a titanium alkoxide compound, an aluminum alkoxide compound, or a zirconium alkoxide compound.

The silicon alkoxide compound preferably includes a silicon alkoxide compound having an oxirane ring, more preferably a silicon alkoxide compound having an aliphatic ring having an oxirane ring fused thereto.

It is also preferred that the silicon alkoxide compound have a group selected from the group consisting of an amino group, an isocyanate group, a thiol group, an ethylenically unsaturated group, and an acid anhydride group.

The oxirane ring, the amino group, the isocyanate group, the thiol group, the ethylenically unsaturated group, and the acid anhydride group are preferably present as or in a substituent on a nonhydrolyzable group (e.g., an alkyl group) of the silicon alkoxide compound.

The titanium alkoxide compound preferably includes an atom of at least one of N, P, or S. It is also preferred that the titanium alkoxide compound have an acetato structure. The aluminum alkoxide compound preferably includes at least one of an acetonato structure or an acetato structure. The zirconium alkoxide compound preferably includes at least one of an acetonato structure, an acetato structure, or a lactato structure.

The metal alkoxide compound serving as the component (c) will now be described in more detail with reference to general formulas.

The metal alkoxide compound serving as the component (c) preferably includes at least one compound represented by general formula (1) or (2) below.

General formula (1): R¹ₘ-M-(OR²)ₙ₋ₘ

General formula (2): O-[M-(OR²)ₙ₋₁]₂

In general formulas (1) and (2), M represents Si, Al, B, Ba, Bi, Ca, Ga, Ge, Hf, In, La, Mg, Nb, P, Sr, Sn, Ta, Ti, V, Y, or Zr. Preferably, M is Si, Ti, Al, or Zr.

R¹ represents a hydrogen atom, an alkyl group, a cycloalkyl group, an acyl group, an aryl group, or an unsaturated aliphatic group.

The alkyl group that can be selected as R¹ may be a linear alkyl group, a branched alkyl group, or an aralkyl group. The alkyl group preferably has 1 to 20 carbon atoms, more preferably 1 to 15 carbon atoms, even more preferably 1 to 10 carbon atoms, particularly preferably 1 to 8 carbon atoms. If the alkyl group is an aralkyl group, it preferably has 7 to 30 carbon atoms. Specific examples of preferred alkyl groups include methyl, ethyl, propyl, isopropyl, n-butyl, s-butyl, isobutyl, t-butyl, n-pentyl, n-hexyl, n-heptyl, n-octyl, n-decyl, n-tridecyl, n-octadecyl, benzyl, and phenethyl.

It is also preferred that the alkyl group that can be selected as R¹ have an oxirane ring. In this case, M is preferably Si. That is, metal alkoxides in which M is Si and R¹ is an epoxyalkyl group are suitable as the component (c). Metal alkoxides in which M is Si and R¹ is an epoxycycloalkylalkyl group are also suitable as the component (c). The cycloalkyl group in the epoxycycloalkylalkyl group (i.e., a cycloalkyl group having a structure in which an oxirane ring is fused thereto) that can be selected as R¹ is preferably 4- to 8-membered, more preferably 5- or 6-membered, even more preferably 6-membered (i.e., an epoxycyclohexyl group).

It is also preferred that the alkyl group that can be selected as R¹ have a group selected from the group consisting of an amino group, an isocyanate group, a thiol group, an ethylenically unsaturated group, and an acid anhydride group.

The cycloalkyl group that can be selected as R¹ preferably has 3 to 20 carbon atoms, more preferably 3 to 15 carbon atoms, more preferably 3 to 10 carbon atoms, particularly preferably 3 to 8 carbon atoms. Specific examples of preferred cycloalkyl groups include cyclopropyl, cyclopentyl, and cyclohexyl.

The acyl group that can be selected as R¹ preferably has 2 to 40 carbon atoms, more preferably 2 to 30 carbon atoms, even more preferably 2 to 20 carbon atoms, particularly preferably 2 to 18 carbon atoms.

The aryl group that can be selected as R¹ preferably has 6 to 20 carbon atoms, more preferably 6 to 15 carbon atoms, even more preferably 6 to 12 carbon atoms, particularly preferably 6 to 10 carbon atoms. Specific examples of preferred aryl groups include phenyl and naphthyl, more preferably phenyl.

The unsaturated aliphatic group that can be selected as R¹ preferably has 1 to 5 carbon-carbon unsaturated bonds, more preferably 1 to 3 carbon-carbon unsaturated bonds, even more preferably 1 or 2 carbon-carbon unsaturated bonds, particularly preferably 1 carbon-carbon unsaturated bond. The unsaturated aliphatic group may include a heteroatom and is also preferably a hydrocarbon group. If the unsaturated aliphatic group is a hydrocarbon group, it preferably has 2 to 20 carbon atoms, more preferably 2 to 15 carbon atoms, even more preferably 2 to 10 carbon atoms, still more preferably 2 to 8 carbon atoms, or preferably 2 to 5 carbon atoms. More preferably, the unsaturated aliphatic group is an alkenyl group or an alkynyl group.

R¹ is preferably a hydrogen atom, an alkyl group, a cycloalkyl group, or aryl group, more preferably an alkyl group or a cycloalkyl group.

If the compound of general formula (1) has two or more R¹ groups, the two R¹ groups may be linked to each other to form a ring.

R² represents a hydrogen atom, an alkyl group, a cycloalkyl group, an acyl group, an alkenyl group, an aryl group, a phosphonate group (phosphonic acid group), or -SO₂R^{S}, where R^{S} represents a substituent.

The alkyl group, the cycloalkyl group, the acyl group, and the aryl group that can be selected as R² have the same meaning as the alkyl group, the cycloalkyl group, the acyl group, and the aryl group, respectively, that can be selected as R¹, and preferred forms thereof are also the same. It is also preferred that the alkyl group that can be selected as R² have an amino group as a substituent.

The alkenyl group that can be selected as R² may be a linear alkenyl group or a branched alkenyl group. The alkenyl group preferably has 2 to 18 carbon atoms, more preferably 2 to 7 carbon atoms, even more preferably 2 to 5 carbon atoms. Specific examples of preferred alkenyl groups include vinyl, allyl, butenyl, pentenyl, and hexenyl. The alkenyl group is preferably a substituted alkenyl group.

The phosphonate group that can be selected as R² is a group represented as -P(=O)(-OR^{P1})OR^{P2}. R^{P1} and R^{P2} represent a hydrogen atom or a substituent. The substituent is preferably an alkyl group or a phosphonate group. The alkyl group that can be selected as R^{P1} and R^{P2} has the same meaning as the alkyl group that can be selected as R¹ described above, and preferred forms thereof are also the same. The phosphonate group that can be selected as R^{P1} and R^{P2} has the same meaning as the phosphonate group that can be selected as R², and preferred forms thereof are also the same. If R^{P1} or R^{P2} is a phosphonate group, the R^{P1} and R^{P2} groups that form the phosphonate group are preferably alkyl groups.

It is preferred that both the R^{P1} and R^{P2} groups of the phosphonate group that can be selected as R² be alkyl groups, or R^{P1} be a hydrogen atom whereas R^{P2} be a phosphonate group.

Phosphonate groups and phosphite groups (phosphorous acid groups) are tautomers of each other; therefore, "phosphonate group" in the present invention is meant to include phosphite groups.

The substituent R^{S} in the -SO₂R^{S} group that can be selected as R² is preferably an alkyl group or an aryl group. Preferred forms of the alkyl and aryl groups that can be selected as R^{S} include the preferred forms of the alkyl and aryl groups, respectively, that can be selected as R¹ described above. In particular, R^{S} is preferably a phenyl group having an alkyl group as a substituent. Preferred forms of the alkyl group are the same as the preferred forms of the alkyl group that can be selected as R¹ described above.

If the compound of general formula (1) or the compound of general formula (2) has two or more R² groups, the two R² groups may be linked to each other to form a ring.

m is an integer of 0 to 3, and n is the valence of M. In addition, n > m is satisfied. Preferably, m is 0 or 1, more preferably 0.

If M is Ti, the compound represented by general formula (1) or (2) above preferably includes an atom of at least one of N, P, or S. If the compound represented by general formula (1) or (2) has N, N is preferably present in the form of an amino group.

If the compound represented by general formula (1) or (2) has P, P is preferably present in the form of a phosphate group (phosphoric acid group) or a phosphonate group (phosphonic acid group).

If the compound represented by general formula (1) or (2) has S, S is preferably present in the form of a sulfonyl group (-SO₂-).

If M is Ti, it is also preferred that the compound represented by general formula (1) or (2) above have an acyl group as R², that is, an acetato structure, described later, as OR².

Ti is typically tetravalent.

If M is Al, at least one OR² group in general formula (1) or (2) above preferably has an acetonato structure. "Acetonato structure" refers to a structure coordinated to M with one hydrogen atom removed from acetone or a compound having a structure in which acetone has a substituent. The ligand atom coordinated to M is typically an oxygen atom. The acetonato structure is preferably a structure that has an acetylacetone structure ("CH₃-C(=O)-CH₂-C(=O)-CH₃") as the basic structure with one hydrogen atom removed therefrom and that is coordinated to M using an oxygen atom as a ligand atom (i.e., an acetylacetonato structure). "Have an acetylacetone structure as the basic structure" above is meant to include the acetylacetone structure and a structure derived from the acetylacetone structure by replacing a hydrogen atom with a substituent. Examples of forms in which M is Al and OR² has an acetonato structure include the compounds A-2 and A-3 described later.

If M is Al, at least one OR² group in general formula (1) or (2) above preferably has an acetato structure. In the present invention, "acetato structure" refers to a structure coordinated to M with one hydrogen atom removed from acetic acid, an acetic acid ester, or a compound having a structure in which acetic acid or an acetic acid ester has a substituent (including forms in which the methyl group of acetic acid has an alkyl group as a substituent).

The ligand atom coordinated to M is typically an oxygen atom. The acetato structure is preferably a structure that has an alkyl acetoacetate structure ("CH₃-C(=O)-CH₂-C(=O)-ORₐₗₖ" (where Rₐₗₖ represents an alkyl group (preferably an alkyl group having 1 to 10 carbon atoms, more preferably an alkyl group having 1 to 4 carbon atoms))) as the basic structure with one hydrogen atom removed therefrom and that is coordinated to M using an oxygen atom as a ligand atom (i.e., an alkyl acetoacetato structure). "Have an alkyl acetoacetate structure as the basic structure" above is meant to include the alkyl acetoacetate structure and a structure derived from the alkyl acetoacetate structure by replacing a hydrogen atom with a substituent. Examples of forms in which M is Al and OR² has an acetato structure include the compounds A-3, A-4, and A-5 described later.

Al is typically trivalent.

If M is Zr, at least one OR² group in general formula (1) or (2) above preferably has an acetonato structure. The acetonato structure has the same meaning as the acetonato structure described in the context of forms in which M is Al. Examples of forms in which M is Zr and OR² has an acetonato structure include the compounds Z-3 and Z-6 described later.

If M is Zr, it is also preferred that at least one OR² group in general formula (1) or (2) above have an acetato structure. The acetato structure has the same meaning as the acetato structure described in the context of forms in which M is Al. Examples of forms in which M is Zr and OR² has an acetato structure include the compound Z-7 described later.

If M is Zr, it is also preferred that at least one OR² group in general formula (1) or (2) above have a lactato structure. "Lactato structure" refers to a structure that has a lactate ion (lactate) as the basic structure with one hydrogen atom removed therefrom and that is coordinated to M. "Have a lactate ion as the basic structure" above is meant to include the lactate ion and a structure derived from the lactate ion by replacing a hydrogen atom with a substituent. The ligand atom coordinated to M is typically an oxygen atom. Examples of forms in which M is Zr and OR² has a lactato structure include the compound Z-4 described later.

If M is Zr, a form is also preferred in which at least one R² group in general formula (1) or (2) above is an acyl group. Examples of forms in which M is Zr and R² is an acyl group include the compound Z-5 described later.

Zr is typically tetravalent.

The above groups that can be selected as R¹ or R² may have, as a substituent, an anionic group having a counter cation (salt-type substituent). "Anionic group" refers to a group capable of forming an anion. An example of an anionic group having a counter cation is a carboxylate ion group having an ammonium ion as a counter cation. In this case, it is sufficient that the counter cation be present in the compound represented by general formula (1) or (2) such that the entire compound has zero charge.

Specific examples of compounds represented by general formula (1) are given below, although they are not intended to limit the present invention.

### Examples in which M is Si

2-(3,4-Epoxycyclohexyl)ethyltrimethoxysilane
5,6-Epoxyhexyltriethoxysilane
3-Glycidoxypropyltrimethoxysilane
3-Aminopropyltrimethoxysilane
3-Isocyanatopropyltriethoxysilane
3-Mercaptopropyltrimethoxysilane
(3-Methacryloxypropyl)trimethoxysilane
3-Trimethoxysilylpropylsuccinic anhydride
Allyltrimethoxysilane
Tetraethoxysilane
Vinyltrimethoxysilane
p-Styryltrimethoxysilane
Tris-(trimethoxysilylpropyl) isocyanurate
3-Ureidopropyltrialkoxysilane
4-Amino-3,3-dimethylbutyltrimethoxysilane
N-(2-Aminoethyl)-3-aminopropyltrimethoxysilane
Bis(3-trimethoxysilylpropyl)amine
3-Acetoxypropyltrimethoxysilane
N-(Hydroxyethyl)-N-methylaminopropyltrimethoxysilane Examples in which M is Ti
Isopropyl triisostearoyl titanate
Isopropyl tridodecylbenzenesulfonyl titanate
Isopropyl trioctanoyl titanate
Isopropyl tri(dioctyl phosphite) titanate
Isopropyl tris(dioctyl pyrophosphate) titanate
Isopropyl tri(dioctyl sulfate) titanate
Isopropyl tricumylphenyl titanate
Isopropyl tri(N-aminoethyl-aminoethyl) titanate
Isopropyl dimethacrylisostearoyl titanate
Isopropyl isostearoyldiacryl titanate
Isobutyl trimethyl titanate
Diisostearoyl ethylene titanate
Diisopropyl bis(dioctyl pyrophosphate) titanate
Dioctyl bis(ditridecyl phosphite) titanate
Dicumylphenyl oxyacetate titanate
Bis(dioctyl pyrophosphate) oxyacetate titanate
Bis(dioctyl pyrophosphate) ethylene titanate
Bis(dioctyl pyrophosphate) oxyacetate titanate
Tetraisopropyl titanate
Tetra-n-butyl titanate
Tetraoctyl titanate
Tetrastearyl titanate
Tetraisopropyl bis(dioctyl phosphite) titanate
Tetraoctyl bis(di-tridecyl phosphite) titanate
Tetra(2,2-diallyloxymethyl-1-butyl) bis(di-tridecyl)phosphite titanate
Butyl titanate dimer
Titanium tetraacetylacetonate
Titanium ethylacetoacetate
Titanium octyleneglycolate
Titanium di-2-ethylhexoxybis(2-ethyl-3-hydroxyhexoxide) Examples in which M is Al
Aluminum triethylate
Aluminum triisopropylate
Aluminum tri-sec-butylate
Aluminum tris(ethylacetoacetate)
Ethylacetoacetate aluminum diisopropylate
Aluminum monoacetylacetonate bis(ethylacetoacetate)
Aluminum tris(acetylacetonate)
Diisopropoxyaluminum-9-octadecenylacetoacetate
Aluminum diisopropoxymonoethylacetoacetate
Aluminum trisethylacetoacetate
Aluminum trisacetylacetonate
Mono-sec-butoxyaluminum diisopropylate
Ethylacetoacetate aluminum diisopropylate
Diethylacetoacetate aluminum isopropylate
Aluminum bisethylacetoacetate monoacetylacetonate
Aluminum trisethylacetoacetate
Aluminum octadecylacetoacetate diisopropylate Examples in which M is Zr
Tetra-n-propoxyzirconium (also known as zirconium tetra-n-propoxide)
Tetra-n-butoxyzirconium (also known as zirconium tetra-n-butoxide)
Zirconium tetraacetylacetonate
Zirconium tributoxymonoacetylacetonate
Zirconium dibutoxybis(acetylacetonate)
Zirconium dibutoxybis(ethylacetoacetate)
Zirconium tributoxyethylacetoacetate
Zirconium monobutoxyacetylacetonate bis(ethylacetoacetate)
Zirconium tributoxymonostearate (also known as zirconium tri-n-butoxide stearate)
Zirconium stearate
Zirconium lactate ammonium salt
Zirconium monoacetylacetonate

An example in which M is B is triethyl borate. An example in which M is Ba is barium acetylacetonate hydrate. An example in which M is Bi is bismuth tri-tert-amyloxide. An example in which M is Ca is calcium tert-butoxide. An example in which M is Ga is gallium triisopropoxide. An example in which M is Ge is germanium tetraethoxide. An example in which M is Hf is hafnium tetra-n-butoxide. An example in which M is In is indium triisopropoxide. An example in which M is La is lanthanum triisopropoxide. An example in which M is Mg is magnesium bis(2-methyl-2-propanolate). An example in which M is Nb is niobium penta-n-butoxide. An example in which M is P is trimethyl phosphate. An example in which M is Sr is strontium isopropoxide. An example in which M is Sn is tin n-butoxide. An example in which M is Ta is tantalum penta-n-butoxide. An example in which M is V is vanadium tri-n-butoxide oxide. An example in which M is Y is yttrium n-butoxide.

In the present invention, "the adhesive includes a metal alkoxide compound as the component (c)" is meant to encompass a state in which the metal alkoxide compound serving as the component (c) is present as-is in the adhesive, a state in which the metal alkoxide compound serving as the component (c) is present in a form hydrolyzed in the adhesive, and a state in which the metal alkoxide compound serving as the component (c) is present in a form hydrolyzed in the adhesive and reacted or interacted with another component such as the epoxy resin or the polyamine compound.

The amount of the component (c) present in the adhesive according to the present invention may be appropriately adjusted depending on the purpose. For example, the amount of the component (c) present in the adhesive may be 0.05 to 30 parts by mass, more preferably 0.1 to 20 parts by mass, even more preferably 0.2 to 10 parts by mass, still more preferably 0.2 to 6 parts by mass, still even more preferably 0.3 to 4 parts by mass, particularly preferably 0.3 to 2 parts by mass, or preferably 0.3 to 1.2 parts by mass, or preferably 0.3 to 1.0 parts by mass, or preferably 0.3 to 0.9 parts by mass, or preferably 0.35 to 0.8 parts by mass, based on 100 parts by mass of the epoxy resin.

### Cured Product

A cured product according to the present invention is a cured product formed by curing the adhesive according to the present invention. Specifically, the cured product according to the present invention is used as a member that forms a bonded region of an endoscope. The curing temperature of the adhesive according to the present invention is not particularly limited and is appropriately adjusted depending on the purpose by taking into account, for example, the heat resistance of the member to be bonded and the curing time. It is preferred to mix the individual components together while removing bubbles. To this end, mixing is typically performed under reduced pressure. The curing temperature is preferably 100°C or lower, more preferably 90°C or lower, even more preferably 80°C or lower. To sufficiently perform the curing reaction, the curing temperature is preferably 0°C or higher, more preferably 10°C or higher. The curing reaction time can be appropriately set depending on the purpose. Typically, the curing reaction is performed for 1.5 to 200 hours to obtain a cured product.

### Endoscope

An endoscope according to the present invention includes a constituent member secured with a cured product according to the present invention. "Constituent member secured with a cured product according to the present invention" means that at least one of the members that form the endoscope is secured to a support member with a cured product according to the present invention therebetween.

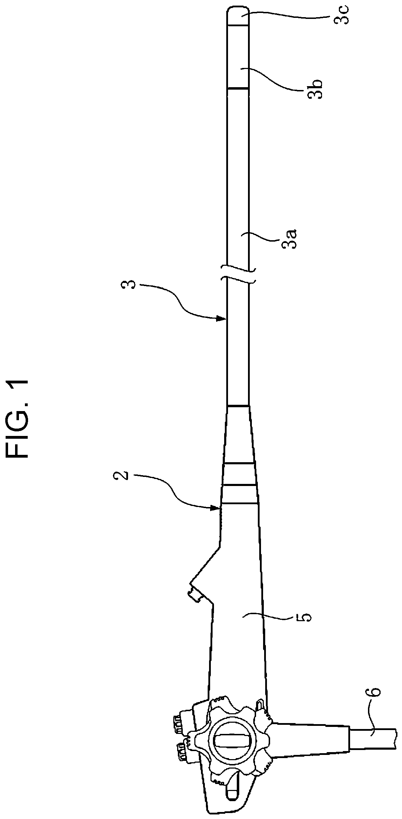

An example of an endoscope (electronic endoscope) according to the present invention will now be described. Electronic endoscopes, which are widely used as medical devices, incorporate a flexible tube for an endoscope (a flexible tube for an endoscope may be hereinafter simply referred to as "flexible tube"). In the example illustrated in FIG. 1, an electronic endoscope 2 is composed of an insertion section 3 for insertion into a body cavity, a main-body operating section 5 connected to the proximal end portion of the insertion section 3, and a universal cord 6 for connection to a processor device and a light source device. The insertion section 3 is composed of a flexible tube 3a connected to the main-body operating section 5, an angle portion 3b connected to the flexible tube 3a, and a tip portion 3c connected to the distal end of the angle portion 3b and composed mainly of a metal (e.g., stainless steel) member. The tip portion 3c has an imaging device (not illustrated) built thereinto for imaging the interior of a body cavity. The flexible tube 3a, which accounts for most of the length of the insertion section 3, is flexible substantially over the entire length thereof. In particular, the portion to be inserted into a site such as a body cavity has a more flexible structure.

In FIG. 1, a plurality of channels (tubes, not illustrated) are formed so as to extend axially through the insertion section 3 from the main-body operating section 5 to the distal end face of the tip portion 3c.

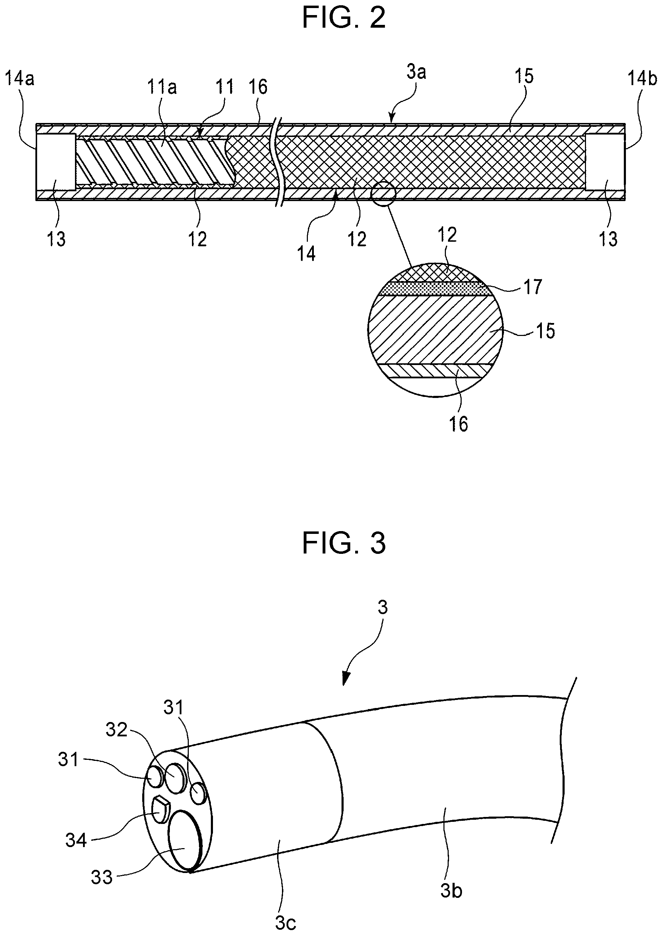

As illustrated in FIG. 2, the flexible tube 3a in FIG. 1 is composed of a flexible tube substrate 14 and a resin layer 15 covering the outer peripheral surface of the flexible tube substrate 14.

Reference numeral 14a denotes the distal side (tip portion 3c side), whereas reference numeral 14b denotes the proximal side (main-body operating section 5 side).

The flexible tube substrate 14 includes a spiral tube 11 disposed on the innermost side and formed by spirally winding a metal strip 11a and a tubular net 12 covering the spiral tube 11 and formed by weaving metal wires. Caps 13 are fitted to both ends of the flexible tube substrate 14. The resin layer 15 is bonded to the flexible tube substrate 14 with an adhesive cured product layer 17 therebetween. The adhesive cured product layer 17 can be formed by applying and curing the adhesive according to the present invention. Although the adhesive cured product layer (bonded region) 17 is illustrated as a layer with uniform thickness for illustration purposes, it does not necessarily have to be in that form, but may be present in irregular form between the resin layer 15 and the flexible tube substrate 14. Rather, the adhesive cured product layer 17 may have negligible thickness, and the resin layer 15 and the flexible tube substrate 14 may be bonded together substantially in contact with each other.

The outer surface of the resin layer 15 is coated with a chemical-resistant coat layer 16 such as one containing fluorine. To clearly illustrate the layer structure, the adhesive cured product layer 17, the resin layer 15, and the coat layer 16 are shown as being thick relative to the diameter of the flexible tube substrate 14.

As illustrated in FIG. 3, illumination windows 31, an observation window 32, and a forceps port 33 are formed in the distal end face of the tip portion 3c. A nozzle 34 for ejecting water and air is also formed in order to clean the distal end face where necessary. The illumination windows 31, the observation window 32, the forceps port 33, and the nozzle 34 are connected to the main-body operating section 5 through the channels.

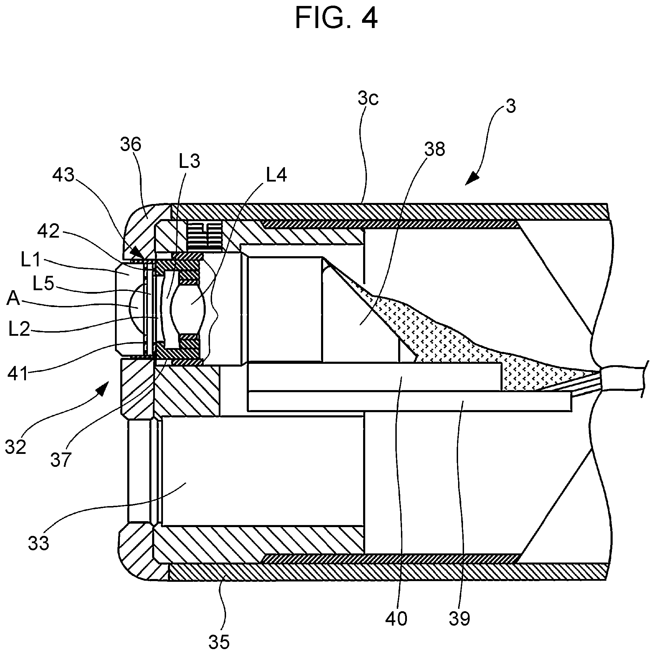

As illustrated in FIG. 4, the tip portion 3c is composed of a tip-portion main body 35 formed of a metal and a distal end cap 36 formed of an electrically insulating material.

An observation unit 43 is an optical system device installed in the observation window 32. The observation unit 43 includes an objective optical system composed of lenses L1 to L5 secured within a lens holder 37 with adhesive cured products 41 and 42. The adhesive cured products 41 and 42 can be formed by applying and curing the adhesive according to the present invention. In the objective optical system, reference character A denotes an air layer. A prism 38 is bonded and secured to the end face of the lens holder 37. The prism 38 bends the optical axis of the objective optical system at a right angle. The prism 38 is secured to a solid-state imaging element 40. The solid-state imaging element 40 is secured to a substrate 39. These members can also be secured by applying the adhesive according to the present invention.

### Method for Manufacturing Endoscope

A method for manufacturing an endoscope according to the present invention is not particularly limited as long as the method includes securing an endoscope constituent member with the adhesive according to the present invention. As for the steps other than securing the endoscope constituent member, common manufacturing steps can be employed to manufacture the endoscope according to the present invention.

The material of the endoscope constituent member to be secured is not particularly limited. Examples of endoscope constituent members include resin members, metal members, and glass members. For example, the endoscope constituent member can be secured to a support member or other member that forms the endoscope by mixing together the individual components to be present in the adhesive according to the present invention, preferably under reduced pressure, injecting or applying the mixture to the area to be bonded, and heating the mixture at -10°C to 60°C (preferably 0°C to 60°C, more preferably 10°C to 50°C) for 1.5 to 200 hours.

The form of use of the adhesive in the method for manufacturing the endoscope according to the present invention will hereinafter be described with reference to the following specific examples, although these examples are not intended to limit the present invention.

Among endoscope constituent members to be secured with the adhesive according to the present invention, an example of a resin member is a tube for insertion into the insertion section of an endoscope. Examples of resin materials that form the tube include fluorocarbon resins such as Teflon (registered trademark), polysulfones, polyesters, polyolefins, and silicones. For example, the adhesive according to the present invention can be used to bond a metal or glass member that forms the insertion section of an endoscope to the tube (to secure a metal or glass member to the tube).

As described above, the adhesive according to the present invention can also be used to form the adhesive cured product layer 17 in FIG. 2. The adhesive according to the present invention can also be used to bond the resin layer 15 to the coat layer 16 in FIG. 2.

The adhesive according to the present invention can be used for outer surface finishing and securing of an end portion of a flexible outer skin tube (resin layer 15) (an end portion on the distal side (angle portion 3b side) of the flexible tube 3a). Specifically, the end portion of the resin layer 15 of the flexible tube 3a is secured to the inner member by binding the end portion from outside with a thread, and the adhesive is then applied and cured so as to cover the thread. If the adhesive according to the present invention forms the outermost layer of the distal end portion of the flexible tube 3a, the thread on the distal end portion is less likely to come undone, and the insertion section can be more easily inserted into a body cavity.

The adhesive according to the present invention can also be used to bond the tip portion 3c to the angle portion 3b and/or to bond the insertion section 3 to the main-body operating section 5. For example, the tip portion 3c is bonded to the angle portion 3b with the adhesive according to the present invention, the bonded region between the tip portion 3c and the angle portion 3b and the nearby region are bound with a thread to reinforce the bonding, and the adhesive is then applied and cured so as to cover the thread. The insertion section 3 can be similarly bonded to the main-body operating section 5.

The adhesive according to the present invention can also be used to secure various tubes for insertion into the insertion section of an endoscope to the tip portion 3c and/or to the main-body operating section 5.

It is also preferred to use the adhesive according to the present invention to seal the illumination windows 31 and the observation window 32 at the tip portion 3c (to secure glass members). A thick coating of the adhesive smoothens the corner of the rim of a lens and can also block light coming from the side of the lens.

The adhesive according to the present invention can also be used to secure members for purposes such as assembling the imaging device to be built into the tip portion 3c, bonding its parts, and sealing the solid-state imaging element 40. The imaging device has an optical system composed of a plurality of optical elements, such as the lenses L1 to L5 and the prism 38, and the solid-state imaging element 40, such as a charge coupled device (CCD), for photoelectric conversion of an optical image formed by the optical system into image signals. The adhesive according to the present invention can be used, for example, to bond together the optical elements such as the lenses L1 to L5 and the prism 38, which are formed of a material such as glass, and to bond the optical elements such as the lenses L1 to L5 and the prism 38 to the substrate 39, which is formed of resin or metal. By such bonding, glass members can be secured, and metal members can also be secured.

The adhesive according to the present invention can also be used to bond, secure, and seal together the solid-state imaging element 40 and the substrate 39. By such bonding, metal members such as those that form a solid-state imaging element and a substrate can be secured.

Thus, the method for manufacturing an endoscope according to the present invention includes a step of securing an endoscope constituent member with the adhesive according to the present invention.

### Examples

The present invention will now be more specifically described based on the following examples, although these examples should not be construed as limiting the present invention. In the examples below, "room temperature" refers to 25°C. In addition, the amount of a component refers to the amount of the component itself; that is, if the raw material includes a solvent, the amount of the component refers to the amount of the component excluding the solvent.

### Preparation Example: Preparation of Adhesives

The components (a) to (c) listed in the following tables were mixed in the ratios (in parts by mass) listed in the following tables. The resulting mixtures were defoamed at room temperature under a reduced pressure of 1.0 Pa with stirring at 2,000 rpm using "Awatori Rentaro ARV-310 (trade name, manufactured by Thinky Corporation)" for 5 minutes to obtain adhesives. In the following Test Examples, the as-prepared adhesives were used.

### Test Examples

### Wet Durability

For each of the adhesives obtained in the Preparation Example, two fluorocarbon rubber sheets (with a length of 30 mm, a width of 25 mm, and a thickness of 5 mm) were obtained and placed on top of each other with the adhesive therebetween such that one longitudinal end of one sheet overlapped one longitudinal end of the other sheet by 3 mm × 25 mm in width. The adhesive was then cured by heating at 80°C for 12 hours to prepare a test specimen. The shear strength (initial) of the test specimen was measured at a tensile speed of 100 mm/min and a temperature of 25°C in accordance with JIS K 6852-1994. A higher shear strength indicates a higher adhesiveness.

In addition, a test specimen prepared in the same manner as above was allowed to stand in an environment at 50°C and 95% RH for 10 days. This high temperature, i.e., 50°C, was intended for accelerated testing. The shear strength (after hygrothermal degradation) of the test specimen was measured in the same manner as above. Shear strength retention (%) was calculated from the following equation. The calculated shear strength retention was evaluated on the following evaluation scale. A higher shear strength retention indicates a higher wet durability. Shear strength retention (%) = (shear strength after hygrothermal degradation/initial shear strength) × 100

### Evaluation Scale

A: The shear strength retention was 80% to less than 100%.
B: The shear strength retention was 60% to less than 80%.
C: The shear strength retention was 40% to less than 60%.
D: The shear strength retention was less than 40%.

The results are summarized in the following tables.

### Hydrogen Peroxide Plasma Sterilization Durability Test

Each of the adhesives obtained in the Preparation Example was poured into a Teflon (registered trademark) mold with a length of 100 mm, a width of 20 mm, and a thickness of 0.4 mm and was allowed to stand at 30°C for 170 hours to obtain a sheet-shaped sample (cured product).

The sheet-shaped sample was subjected to hydrogen peroxide plasma sterilization treatment at room temperature using the advanced course of STERRAD (registered trademark) NX (manufactured by Johnson & Johnson). A sheet-shaped sample (I) before sterilization treatment and a sheet-shaped sample (II) repeatedly subjected to hydrogen peroxide plasma sterilization treatment 100 times were subjected as test specimens to a tensile test in which they were pulled at a tensile speed of 20 mm/min and a gauge length of 20 mm in the longitudinal direction using Autograph AGS-X (trade name, manufactured by Shimadzu Corporation).

Breaking strength retention was defined as the proportion of the breaking strength of the sheet-shaped sample (II) to the breaking strength of the sheet-shaped sample (I) (100 × (breaking strength of sheet-shaped sample (II))/(breaking strength of sheet-shaped sample (I))) and was evaluated for sterilization treatment durability on the following evaluation scale.

### Evaluation Scale

AA: The breaking strength retention was 90% or more.
A: The breaking strength retention was 80% to less than 90%.
B: The breaking strength retention was 60% to less than 80%.
C: The breaking strength retention was 40% to less than 60%.
D: The breaking strength was less than 40% of that before the sterilization treatment, or it was impossible to perform the tensile test because the sample was degraded and damaged during the hydrogen peroxide plasma sterilization treatment.

The results are summarized in the following tables.

**Table 1**

| | | | Example 1 | Example 2 | Example 3 | Example 4 | Example 5 | Example 6 | Example 7 | Example 8 | Example 9 | Example 10 |
|---|---|---|---|---|---|---|---|---|---|---|---|---|
| Component (a) | Epoxy resin | Type | (a-1) | (a-2) | (a-3) | (a-4) | (a-5) | (a-1) | (a-1) | (a-1) | (a-1) | (a-1) |
| | | Amount | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-2) | (b-3) | (b-1) | (b-1) | (b-1) |
| | | Amount | 25 | 25 | 25 | 25 | 25 | 25 | 25 | 25 | 25 | 25 |
| Component (c) | Metal alkoxide | Type | (c-1) | (c-1) | (c-1) | (c-1) | (c-1) | (c-1) | (c-1) | (c-2) | (c-3) | (c-4) |
| | | Metal species | Si | Si | Si | Si | Si | Si | Si | Si | Si | Si |
| | | Amount | 0.5 | 0.5 | 0.5 | 0.5 | 0.5 | 0.5 | 0.5 | 0.5 | 0.5 | 0.5 |
| Wet durability | | | A | A | A | A | A | A | A | C | C | A |
| Sterilization durability | | | AA | AA | AA | A | B | A | A | A | A | A |

**Table 2**

| | | | Example 11 | Example 12 | Example 13 | Example 14 | Example 15 | Example 16 | Example 17 | Example 18 | Example 19 | Example 20 |
|---|---|---|---|---|---|---|---|---|---|---|---|---|
| Component (a) | Epoxy resin | Type | (a-1) | (a-1) | (a-1) | (a-1) | (a-1) | (a-1) | (a-1) | (a-1) | (a-1) | (a-1) |
| | | Amount | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-1) | (b-1) | (b-1) | (b-1) | (b-1) |
| | | Amount | 25 | 25 | 25 | 25 | 25 | 25 | 25 | 25 | 25 | 25 |
| Component (c) | Metal alkoxide | Type | (c-5) | (c-6) | (c-7) | (c-8) | (c-9) | (c-10) | (c-11) | (c-12) | (c-13) | (c-14) |
| | | Metal species | Si | Si | Si | Si | Si | Si | Ti | Ti | Ti | Ti |
| | | Amount | 0.5 | 0.5 | 0.5 | 0.5 | 0.5 | 0.5 | 0.5 | 0.5 | 0.5 | 0.5 |
| Wet durability | | | A | B | B | A | B | C | C | C | B | A |
| Sterilization durability | | | A | A | A | A | A | B | B | B | A | A |

**Table 3**

| | | | Example 21 | Example 22 | Example 23 | Example 24 | Example 25 | Example 26 | Example 27 | Example 28 | Example 29 | Example 30 |
|---|---|---|---|---|---|---|---|---|---|---|---|---|
| Component (a) | Epoxy resin | Type | (a-1) | (a-1) | (a-1) | (a-1) | (a-1) | (a-1) | (a-1) | (a-1) | (a-1) | (a-1) |
| | | Amount | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-1) | (b-1) | (b-1) | (b-1) | (b-1) |
| | | Amount | 25 | 25 | 25 | 25 | 25 | 25 | 25 | 25 | 25 | 25 |
| Component (c) | Metal alkoxide | Type | (c-15) | (c-16) | (c-17) | (c-18) | (c-19) | (c-20) | (c-21) | (c-22) | (c-23) | (c-24) |
| | | Metal species | Ti | Ti | Ti | Ti | Ti | Ti | Al | Al | Al | Al |
| | | Amount | 0.5 | 0.5 | 0.5 | 0.5 | 0.5 | 0.5 | 0.5 | 0.5 | 0.5 | 0.5 |
| Wet durability | | | A | A | A | A | A | C | C | A | A | A |
| Sterilization durability | | | A | A | A | A | A | B | B | AA | AA | AA |

**Table 4**

| | | | Example 31 | Example 32 | Example 33 | Example 34 | Example 35 | Example 36 | Example 37 | Example 38 | Example 39 | Example 40 |
|---|---|---|---|---|---|---|---|---|---|---|---|---|
| Component (a) | Epoxy resin | Type | (a-1) | (a-1) | (a-1) | (a-1) | (a-1) | (a-1) | (a-1) | (a-1) | (a-1) | (a-1) |
| | | Amount | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-1) | (b-1) | (b-1) | (b-1) | (b-1) |
| | | Amount | 25 | 25 | 25 | 25 | 25 | 25 | 25 | 25 | 25 | 25 |
| Component (c) | Metal alkoxide | Type | (c-25) | (c-26) | (c-27) | (c-28) | (c-29) | (c-30) | (c-31) | (c-32) | (c-1) | (c-1) |
| | | Metal species | Al | Zr | Zr | Zr | Zr | Zr | Zr | Zr | Si | Si |
| | | Amount | 0.5 | 0.5 | 0.5 | 0.5 | 0.5 | 0.5 | 0.5 | 0.5 | 0.1 | 0.3 |
| Wet durability | | | A | C | C | A | A | A | A | A | C | A |
| Sterilization durability | | | A | B | B | A | A | A | A | A | AA | AA |

**Table 5**

| | | | Example 41 | Example 42 | Example 43 | Example 44 | Example 45 | Example 46 | Example 47 | Example 48 | Example 49 |
|---|---|---|---|---|---|---|---|---|---|---|---|
| Component (a) | Epoxy resin | Type | (a-1) | (a-1) | (a-1) | (a-1) | (a-1) | (a-1) | (a-1) | (a-1) | (a-1) |
| | | Amount | 100 | 100 | 100 | 100 | 100 | 100 | 100 | 100 | 100 |
| Component (b) | Polyamine | Type | (b-1) | (b-1) | (b-1) | (b-1) | (b-1) | (b-1) | (b-1) | (b-1) | (b-1) |
| | | Amount | 25 | 25 | 25 | 25 | 25 | 25 | 25 | 25 | 25 |
| Component (c) | Metal alkoxide | Type | (c-1) | (c-1) | (c-1) | (c-14) | (c-14) | (c-22) | (c-22) | (c-28) | (c-28) |
| | | Metal species | Si | Si | Si | Ti | Ti | Al | Al | Zr | Zr |
| | | Amount | 1.0 | 1.5 | 2.0 | 0.1 | 1.0 | 0.1 | 1.0 | 0.1 | 1.0 |
| Wet durability | | | A | B | B | C | B | C | B | C | B |
| Sterilization durability | | | A | A | A | A | A | A | A | A | A |

**Table 6**

| | | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 |
|---|---|---|---|---|---|---|---|---|---|---|---|---|---|---|
| Component (a) | Epoxy resin | Type | (a-1) | (a-2) | (a-3) | (a-4) | (a-5) | (a-1) | (a-1) | (a-1) | (a-1) | (a-1) | (a-1) | (a-1) |
| | | Amount | 100 | 100 | 100 | 100 | 100 | 100 | 100 | 100 | 100 | 100 | 100 | 100 |
| Component (b) | Polyamine | Type | (b-1) | (b-1) | (b-1) | (b-1) | (b-1) | (b-4) | (b-4) | (b-5) | (b-6) | (b-7) | (b-8) | (b-9) |
| | | Amount | 25 | 25 | 25 | 25 | 25 | 25 | 25 | 25 | 25 | 25 | 25 | 25 |
| Component (c) | Metal alkoxide | Type | | | | | | | (c-1) | (c-1) | (c-1) | (c-1) | (c-1) | (c-1) |
| | | Metal species | | | | | | | Si | Si | Si | Si | Si | Si |
| | | Amount | | | | | | | 0.5 | 0.5 | 0.5 | 0.5 | 0.5 | 0.5 |
| Wet durability | | | D | D | D | D | D | D | A | A | B | A | A | A |
| Sterilization durability | | | A | A | A | B | C | D | D | D | D | D | D | D |

### Component (a): Epoxy Resin

(a-1):
   Bisphenol A diglycidyl ether (trade name "jER 825", manufactured by Mitsubishi Chemical Corporation, epoxy equivalent weight = 170)
(a-2):
   Bisphenol A diglycidyl ether (trade name "jER 828", manufactured by Mitsubishi Chemical Corporation, epoxy equivalent weight = 190)
(a-3):
   Bisphenol A diglycidyl ether (trade name "jER 834", manufactured by Mitsubishi Chemical Corporation, epoxy equivalent weight = 230)
(a-4):
   Bisphenol F diglycidyl ether (trade name "EPICLON 830", manufactured by DIC Corporation, epoxy equivalent weight = 170)
(a-5):
   Epoxy novolac resin (Product No. 406775, manufactured by Sigma-Aldrich Co., epoxy equivalent weight = 170)

### Component (b): Polyamine

(b-1):
   Polyoxyalkylenetriamine (trade name: T403, manufactured by Mitsui Fine Chemicals, Inc, active hydrogen equivalent weight = 73)
(b-2):
   Polyoxyalkylenediamine (trade name: D400, manufactured by Mitsui Fine Chemicals, Inc, active hydrogen equivalent weight = 100)
(b-3):
   Polyoxyalkylenediamine (trade name: D2000, manufactured by Mitsui Fine Chemicals, Inc, active hydrogen equivalent weight = 500)
(b-4):
   Polyamide-amine (trade name: ST13, manufactured by Mitsubishi Chemical Corporation)
(b-5):
   m-Xylenediamine (trade name: MXDA, manufactured by Mitsubishi Gas Chemical Company, Inc.)
(b-6):
   Tetraethylenepentamine (manufactured by Tokyo Chemical Industry Co., Ltd.)
(b-7):
   1,3-Bisaminomethylcyclohexane (trade name: 1,3-BAC, manufactured by Mitsubishi Gas Chemical Company, Inc.)
(b-8):
   Isophoronediamine (manufactured by Tokyo Chemical Industry Co., Ltd.)
(b-9):
   m-Phenylenediamine (manufactured by Tokyo Chemical Industry Co., Ltd.)

### Component (c): Metal Alkoxide

### Silicon Alkoxide

(c-1):
   2-(3,4-Epoxycyclohexyl)ethyltrimethoxysilane (trade name: KBM-303, manufactured by manufactured by Shin-Etsu Chemical Co., Ltd.)
(c-2):
   5,6-Epoxyhexyltriethoxysilane (trade name: SIE4675.0, manufactured by Gelest, Inc.)
(c-3):
   3-Glycidoxypropyltrimethoxysilane (trade name: KBM-403, manufactured by Shin-Etsu Chemical Co., Ltd.)
(c-4):
   3-Aminopropyltrimethoxysilane (trade name: KBM-903, manufactured by Shin-Etsu Chemical Co., Ltd.)
(c-5):
   3-Isocyanatopropyltriethoxysilane (trade name: KBM-9007N, manufactured by Shin-Etsu Chemical Co., Ltd.)
(c-6):
   3-Mercaptopropyltrimethoxysilane (trade name: KBM-803, manufactured by Shin-Etsu Chemical Co., Ltd.)
(c-7):
   (3-Methacryloxypropyl)trimethoxysilane (trade name: KBM-503, manufactured by Shin-Etsu Chemical Co., Ltd.)
(c-8):
   3-Trimethoxysilylpropylsuccinic anhydride (trade name: X-12-967C, manufactured by Shin-Etsu Chemical Co., Ltd.)
(c-9):
   Allyltrimethoxysilane (trade name: SIA0540.0, manufactured by Gelest, Inc.)
(c-10):
   Tetraethoxysilane (manufactured by Tokyo Chemical Industry Co., Ltd.)
   Titanium Alkoxide
(c-11):
   Tetra-n-butyl titanate (trade name: ORGATIX TA-21, manufactured by Matsumoto Fine Chemical Co. Ltd., T-1 below) 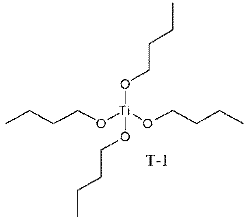
(c-12):
   n-Butyl titanate dimer (trade name: ORGATIX TA-23, manufactured by Matsumoto Fine Chemical Co. Ltd., T-2 below) 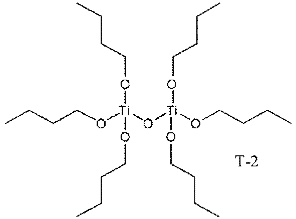
(c-13):
   Isopropyl triisostearoyl titanate (trade name: PLENACT TTS, manufactured by Ajinomoto Fine-Techno Co., Inc., T-3 below) 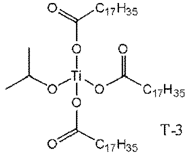
(c-14):
   Dioctyl bis(ditridecyl) phosphate titanate (trade name: PLENACT 46B, manufactured by Ajinomoto Fine-Techno Co., Inc., T-4) 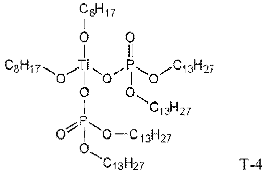
(c-15):
   Diisopropyl bis(dioctyl pyrophosphate) titanate (trade name: PLENACT 38S, manufactured by Ajinomoto Fine-Techno Co., Inc., T-5 below) 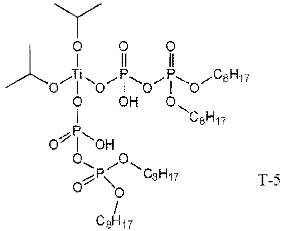
(c-16):
   Bis(dioctyl pyrophosphate) oxyacetate titanate (trade name: PLENACT 138S, manufactured by Ajinomoto Fine-Techno Co., Inc., T-6 below) 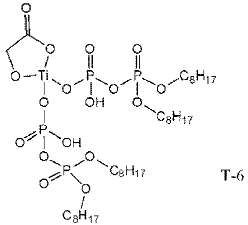
(c-17):
   Bis(dioctyl pyrophosphate) ethylene titanate (trade name: PLENACT 238S, manufactured by Ajinomoto Fine-Techno Co., Inc., T-7 below) 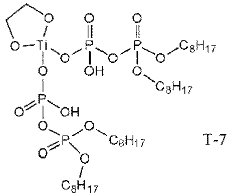
(c-18):
   Isopropyl tri(N-aminoethyl-aminoethyl) titanate (trade name: PLENACT 44, manufactured by Ajinomoto Fine-Techno Co., Inc., T-8 below) 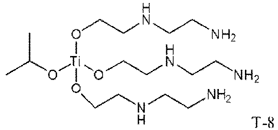
(c-19):
   Isopropyl tridodecylbenzenesulfonyl titanate (trade name: PLENACT 9SA, manufactured by Ajinomoto Fine-Techno Co., Inc., T-9 below) 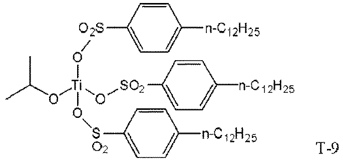
(c-20):
   Titanium di-2-ethylhexoxybis(2-ethyl-3-hydroxyhexoxide) (trade name: ORGATIX TC-201, manufactured by Matsumoto Fine Chemical Co. Ltd., T-10 below) 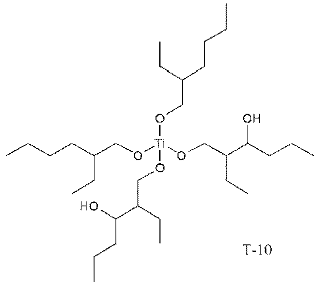
(c-21):
   Aluminum sec-butoxide (trade name: ASBD, manufactured by Kawaken Fine Chemicals Co., Ltd., A-1 below) 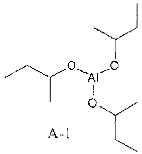
(c-22):
   Aluminum trisacetylacetonate (trade name: ORGATIX AL-3100, manufactured by Matsumoto Fine Chemical Co. Ltd., A-2 below) 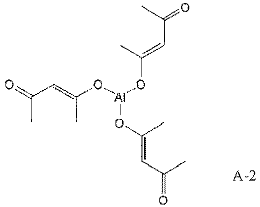
(c-23):
   Aluminum bisethylacetoacetate monoacetylacetonate (trade name: ORGATIX AL-3200, manufactured by Matsumoto Fine Chemical Co. Ltd., A-3 below) 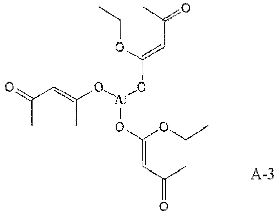
(c-24):
   Aluminum trisethylacetoacetate (trade name: ORGATIX AL-3215, manufactured by Matsumoto Fine Chemical Co. Ltd., A-4 below) 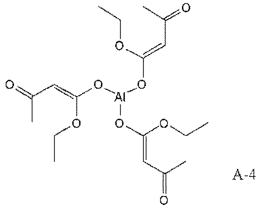
(c-25):
   Aluminum octadecylacetoacetate diisopropylate (trade name: PLENACT AL-M, manufactured by Ajinomoto Fine-Techno Co., Inc., A-5) 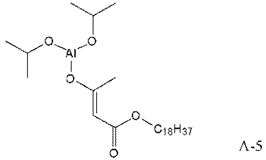
(c-26):
   Zirconium tetra-n-propoxide (trade name: ORGATIX ZA-45, manufactured by Matsumoto Fine Chemical Co. Ltd., Z-1 below) 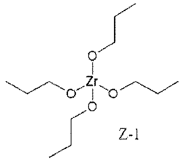
(c-27):
   Zirconium tetra-n-butoxide (trade name: ORGATIX ZA-65, manufactured by Matsumoto Fine Chemical Co. Ltd., Z-2 below) 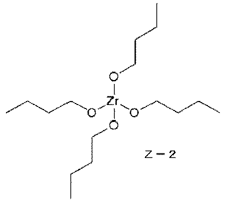
(c-28):
   Zirconium tetraacetylacetonate (trade name: ORGATIX ZC-150, manufactured by Matsumoto Fine Chemical Co. Ltd., Z-3 below) 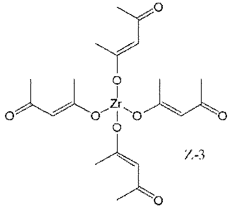
(c-29):
   Zirconium lactate ammonium salt (trade name: ORGATIX ZC-300, manufactured by Matsumoto Fine Chemical Co. Ltd., Z-4 below) 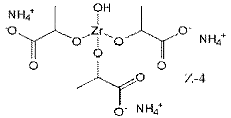
(c-30):
   Zirconium tri-n-butoxide stearate (trade name: ORGATIX ZC-320, manufactured by Matsumoto Fine Chemical Co. Ltd., Z-5 below) 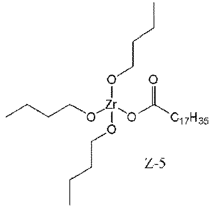
(c-31):
   Zirconium tri-n-butoxymonoacetylacetonate (trade name: ORGATIX ZC-540, manufactured by Matsumoto Fine Chemical Co. Ltd., Z-6 below) 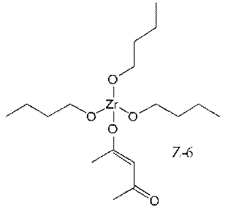
(c-32):
   Zirconium di-n-butoxybis(ethylacetoacetate) (trade name: ORGATIX ZC-580, manufactured by Matsumoto Fine Chemical Co. Ltd., Z-7 below)

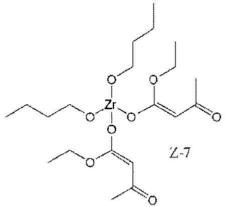

As shown in the tables, the cured products obtained from the adhesives in which an epoxy resin serving as the component (a) and a polyamine compound serving as the component (b) were used in combination as adhesive components but which contained no component (c) exhibited low wet durability (Comparative Examples 1 to 5). In addition, the cured products obtained from the adhesives in which the structure of the polyamine compound did not satisfy the requirement regarding the component (b) exhibited low sterilization durability (Comparative Examples 6 to 12).

In contrast, the adhesives containing all components (a) to (c) exhibited less degradation after extended exposure to wet conditions or after repeated exposure to sterilization treatment (Examples 1 to 49).

While the present invention has been described in conjunction with embodiments thereof, we do not intend to limit our invention in any detail of the description unless otherwise specified. Rather, we believe that the invention should be broadly construed without departing from the scope of the invention as defined by the appended claims.

This application claims priority from JP2019-032974 filed in Japan on February 26, 2019.

### Reference Signs List

- 2: electronic endoscope (endoscope)

- 3: insertion section
- 3a: flexible tube
- 3b: angle portion
- 3c: tip portion
- 5: main-body operating section
- 6: universal cord
- 11: spiral tube
- 11a: metal strip
- 12: tubular net
- 13: cap
- 14: flexible tube substrate
- 14a: distal side
- 14b: proximal side
- 15: resin layer
- 16: coat layer
- 17: adhesive cured product layer
- 31: illumination window
- 32: observation window
- 33: forceps port
- 34: nozzle
- 35: tip-portion main body
- 36: distal end cap
- 37: lens holder
- 38: prism
- 39: substrate
- 40: solid-state imaging element
- 41: adhesive cured product
- 42: adhesive cured product
- 43: observation unit

- L1 to L5: lens

## Claims

1. An adhesive for an endoscope, comprising the following (a) to (c):
(a) an epoxy resin including at least one of a bisphenol A epoxy resin, a bisphenol F epoxy resin, or a phenol novolac epoxy resin;
(b) a polyamine compound having an oxygen atom but no amide bond in a molecule thereof; and
(c) a metal alkoxide compound, wherein the polyamine compound has an oxyalkylene structure.

2. The adhesive for an endoscope according to claim 1, wherein the adhesive for an endoscope comprises a silicon alkoxide compound as the metal alkoxide compound.

3. The adhesive for an endoscope according to any one of claims 1 to 2, wherein the adhesive for an endoscope comprises a silicon alkoxide compound having an oxirane ring as the metal alkoxide compound.

4. The adhesive for an endoscope according to any one of claims 1 to 3, wherein the adhesive for an endoscope comprises, as the metal alkoxide compound, a silicon alkoxide compound having an aliphatic ring having an oxirane ring fused thereto.

5. The adhesive for an endoscope according to any one of claims 1 to 4, wherein the adhesive for an endoscope comprises, as the metal alkoxide compound, at least one of a titanium alkoxide compound, an aluminum alkoxide compound, or a zirconium alkoxide compound.

6. The adhesive for an endoscope according to claim 5, wherein the titanium alkoxide compound includes an atom of at least one of N, P, or S or has an acetato structure.

7. The adhesive for an endoscope according to claim 5, wherein the aluminum alkoxide compound includes at least one of an acetonato structure or an acetato structure.

8. The adhesive for an endoscope according to claim 5, wherein the zirconium alkoxide compound includes at least one of an acetonato structure, an acetato structure, or a lactato structure.

9. A cured product obtained by curing the adhesive for an endoscope according to any one of claims 1 to 8.

10. An endoscope comprising a constituent member secured with the cured product according to claim 9.

11. A method for manufacturing an endoscope, comprising securing a constituent member with the adhesive for an endoscope according to any one of claims 1 to 8.

## Patentansprüche

1. Klebstoff für ein Endoskop, der die folgenden (a) bis (c) umfasst:
(a) ein Epoxidharz, das mindestens eines von einem Bisphenol-A-Epoxidharz, einem Bisphenol-F-Epoxidharz und einem Phenol-Novolac-Epoxidharz enthält;
(b) eine Polyaminverbindung, die ein Sauerstoffatom, aber keine Amidbindung in einem Molekül davon aufweist; und
(c) eine Metallalkoxidverbindung, wobei die Polyaminverbindung eine Oxyalkylenstruktur aufweist.

2. Klebstoff für ein Endoskop nach Anspruch 1, wobei der Klebstoff für ein Endoskop eine Siliciumalkoxidverbindung als die Metallalkoxidverbindung umfasst.

3. Klebstoff für ein Endoskop nach einem der Ansprüche 1 bis 2, wobei der Klebstoff für ein Endoskop eine Silikonalkoxidverbindung, die einen Oxiranring als die Metallalkoxidverbindung aufweist, umfasst.

4. Klebstoff für ein Endoskop nach einem der Ansprüche 1 bis 3, wobei der Klebstoff für ein Endoskop als die Metallalkoxidverbindung eine Siliciumalkoxidverbindung, die einen aliphatischen Ring mit einem Oxiranring, der damit fusioniert ist, aufweist, umfasst.

5. Klebstoff für ein Endoskop nach einem der Ansprüche 1 bis 4, wobei der Klebstoff für ein Endoskop als die Metallalkoxidverbindung mindestens eine von einer Titanalkoxidverbindung, einer Aluminiumalkoxidverbindung und einer Zirkoniumalkoxidverbindung umfasst.

6. Klebstoff für ein Endoskop nach Anspruch 5, wobei die Titanalkoxidverbindung ein Atom von mindestens einem von N, P und S enthält oder eine Acetatstruktur aufweist.

7. Klebstoff für ein Endoskop nach Anspruch 5, wobei die Aluminiumalkoxidverbindung mindestens eine von einer Acetonatstruktur und einer Acetatstruktur enthält.

8. Klebstoff für ein Endoskop nach Anspruch 5, wobei die Zirkoniumalkoxidverbindung mindestens eine von einer Acetonatstruktur, einer Acetatstruktur und einer Lactatstruktur enthält.

9. Gehärtetes Produkt, das durch Härtung des Klebstoffs für ein Endoskop nach einem der Ansprüche 1 bis 8 erhalten wird.

10. Endoskop, das einen Bestandteil, der mit dem gehärteten Produkt nach Anspruch 9 gesichert ist, umfasst.

11. Verfahren zum Herstellen eines Endoskops, das Sichern eines Bestandteils mit dem Klebstoff für ein Endoskop nach einem der Ansprüche 1 bis 8 umfasst.

## Revendications

1. Adhésif pour un endoscope, comprenant les éléments suivants (a) à (c) :
(a) une résine époxy incluant au moins l'une d'une résine époxy de bisphénol A, d'une résine époxy de bisphénol F ou d'une résine époxy phénol novolaque ;
(b) un composé de polyamine ayant un atome d'oxygène mais aucune liaison amide dans une molécule de celui-ci ; et
(c) un composé d'alkoxyde métallique, dans lequel le composé de polyamine a une structure oxyalkylène.

2. Adhésif pour un endoscope selon la revendication 1, dans lequel l'adhésif pour un endoscope comprend un composé d'alkoxyde de silicium en tant que composé d'alkoxyde métallique.

3. Adhésif pour un endoscope selon l'une quelconque des revendications 1 à 2, dans lequel l'adhésif pour un endoscope comprend un composé d'alkoxyde de silicium ayant un cycle oxirane en tant que composé d'alkoxyde métallique.

4. Adhésif pour un endoscope selon l'une quelconque des revendications 1 à 3, dans lequel l'adhésif pour un endoscope comprend, en tant que composé d'alkoxyde métallique, un composé d'alkoxyde de silicium ayant un cycle aliphatique auquel est fusionné un cycle oxirane.

5. Adhésif pour un endoscope selon l'une quelconque des revendications 1 à 4, dans lequel l'adhésif pour un endoscope comprend, en tant que composé d'alkoxyde métallique, au moins l'un d'un composé d'alkoxyde de titane, d'un composé d'alkoxyde d'aluminium ou d'un composé d'alkoxyde de zirconium.

6. Adhésif pour un endoscope selon la revendication 5, dans lequel le composé d'alkoxyde de titane inclut un atome d'au moins l'un de N, P ou S ou a une structure acétato.

7. Adhésif pour un endoscope selon la revendication 5, dans lequel le composé d'alkoxyde d'aluminium inclut au moins l'une d'une structure acétonato et d'une structure acétato.

8. Adhésif pour un endoscope selon la revendication 5, dans lequel le composé d'alkoxyde de zirconium inclut au moins l'une d'une structure acétonato, d'une structure acétato ou d'une structure lactato.

9. Produit durci obtenu par durcissement de l'adhésif pour un endoscope selon l'une quelconque des revendications 1 à 8.

10. Endoscope comprenant un élément constitutif fixé avec le produit durci selon la revendication 9.

11. Procédé de fabrication d'un endoscope, comprenant fixer un élément constitutif avec l'adhésif pour un endoscope selon l'une quelconque des revendications 1 à 8.
